(19)

Europäisches
Patentamt
European
Patent Office
Office européen
des brevets

(11) **EP 3 281 644 A1**

(12) **EUROPEAN PATENT APPLICATION**

(43) Date of publication:
**14.02.2018 Bulletin 2018/07**

(51) Int Cl.:
***A61K 49/22*** *(2006.01)*

(21) Application number: **16382392.5**

(22) Date of filing: **08.08.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicants:
• **Consejo Superior De Investigaciones Científicas**
  **28006 Madrid (ES)**
• **Università degli Studi di Parma**
  **43121 Parma (IT)**

(72) Inventors:
• **VENTOSA RULL, Nora**
  **08193 BELLATERRA (ES)**

• **VECIANA MIRO, Jaume**
  **08193 BELLATERRA (ES)**
• **ARDIZZONE, Antonio**
  **08193 BELLATERRA (ES)**
• **PAINELLI, Anna**
  **43124 PARMA (IT)**
• **KURHUZENKAU, Siarhei**
  **43124 PARMA (IT)**
• **SISSA, Cristina**
  **43124 PARMA (IT)**

(74) Representative: **Oficina Ponti, SLP**
  **C. de Consell de Cent 322**
  **08007 Barcelona (ES)**

(54) **STABLE FLUORESCENT NANOVESICLES, METHOD FOR OBTAINING THEM AND USES THEREOF**

(57)    The invention provides a stable fluorescent nanovesicles, and particularly a new fluorescent nanovesicle maintaining the fluorescence's property for at least two months in an aqueous media.

The invention is also directed to their uses as a fluorescent probe and to a method for preparing the stable fluorescent nanovesicles in an easy and feasibly way.

FIG 11

EP 3 281 644 A1

## Description

### Field of the invention

The present invention relates to stable fluorescent nanovesicles. In particular, the present invention relates to new fluorescent nanovesicles capable of showing the fluorescence's property for at least two months in an aqueous media.

The present invention also relates to a method for preparing the stable fluorescent nanovesicles, as well as to their uses as fluorescent probes in an aqueous media.

### Background of the invention

Liposomes made with phospholipids are among the most-studied self-assembled nano-objects. They are vesicles composed of one or more concentric lipid bilayers, which separate a small-enclosed liquid compartment, the lumen, from its surroundings.

Particularly, nanoscopic vesicles, composed of sterols and quaternary ammonium surfactants, have been prepared by a compressed fluid-based methodology. The internalization of hydrosoluble proteins within the small enclosed compartment of these nanoscopic vesicles, that is, within their lumen, protects the activity of the protein up to their deliverance, see "Multifunctional Nanovesicle-bioactive conjugates prepared by a one-step scalable method using CO2-expanded solvents" of Nano letters, ACS Publications, July 5, 2013.

EP0185680 discloses methods and compositions for the entrapment of water-soluble compounds, partially water-soluble compounds, or water-insoluble compounds in liposomes composed of salt forms of organic acid derivatives of sterols that are capable of forming bilayers. The sterol liposomes according to this patent can be prepared with or without the use of organic solvents, and different ways for compound entrapment are also described.

Despite versatility of the known vesicles, the stability of these structures is kinetically limited because their lipid building blocks are highly insoluble, and therefore, the collapsed planar lamella is the equilibrium state of aggregation.

On the other side, molecular fluorescent dyes have been also studied for fluorescence microscopy. It is known that small organic molecules can be highly fluorescent. However, they suffer drawbacks such as a low photostability, preventing long time imaging, low biocompatibility and high toxicity [see, Liu, P., Liu, P., Zhao, K. & Li, L. "Photostability enhancement of azoic dyes adsorbed and intercalated into Mg-Al-layered double hydroxide". Opt. Laser Technol. 74, 23-28 (2015)], and in some cases they have a poor solubility in aqueous medium, which limits their use for *in vitro* and *in vivo* applications.

Moreover, inorganic nanostructures with size-dependent optical properties, such as quantum dots (QDs), offer an interesting alternative thanks to their high brightness and photostability. Nevertheless, they require a surface functionalization for reducing the toxicity, improving biocompatibility and attaining site-specificity.

Still another possible alternative are the fluorescent organic nanoparticles (FONs) which are photostable and biocompatible and have a good brightness, due to the high concentration of dyes molecules in a small volume. However, they do not allow the use of water-soluble dyes in aqueous media.

Although, nanovesicles loaded with cationic probes such as rhodamine 6G perchlorate (R6G) have been studied in a vesicle bilayer composed by cholesterol-cetyltrimethylammonium bromide (CTAB) by Ghosh et al. "How Does the Surface Charge of Ionic Surfactant and Cholesterol Forming Vesicles Control Rotational and Translational Motion of Rhodamine 6G Perchlorate (R6G ClO4)" from Langmuir (2015), 31 (8), 2310-2320, such studies reveal that the hydrophobicity and electrostatic repulsion induce the migration of R6G from the vesicle bilayer to the aqueous phase.

Therefore, there is still the need to provide a new structure for use as a fluorescent probe, where fluorescent dyes can be introduced, irrespective of their solubility, and that maintains good fluorescence properties, while having a good chemical and kinetical stability in an aqueous medium, overcoming among others the problems of aggregation and migration of the dye to the aqueous phase.

### Summary of the invention

The present invention was made in view of the prior art described above, and the first object of the present invention is to provide a fluorescent nanovesicle with improved properties, as well as their use for particular applications, which derive from the improved fluorescent structures mentioned above.

To solve the problem, the present invention provides in a first aspect, a stable fluorescent nanovesicle comprising a bilayer membrane having hydrophobic and hydrophilic regions and at least an organic dye that interacts with the hydrophobic and/or the hydrophilic regions of the bilayer membrane, wherein the bilayer membrane comprises a sterol molecule or derivatives thereof assembled with a quaternary ammonium surfactant molecule, and wherein the fluorescent nanovesicle has a fluorescence stability for at least two months in an aqueous media.

Surprisingly, the fluorescent nanovesicle designed by the present inventors provides a new scaffold for nanostructuring dyes in water media, in which the chemical and colloidal stability (physical stability) of the dye at the scaffold is maintained over time. Moreover, the absorbance and emission spectra of the fluorescent nanovesicle are also maintained for at least two months.

Advantageously, a fluorescent nanovesicle is provided whose fluorescence is stable for at least two months in aqueous media and having chemical and physical stability in aqueous media for ever a longer time.

Therefore, according to the first aspect of the present invention, the term "stable" in the sentence "stable fluorescent nanovesicle" includes two stability effects, that is, on one side, it refers to the fluorescence stability of the dye in aqueous media upon light irradiation and, on the other side, it refers to the chemical and physical stability of the nanovesicle containing the dye in aqueous media, thereby providing a stable fluorescent nanovesicle in an aqueous media suitable for use as a fluorescent probe in aqueous media.

Advantageously, the present inventors found that a fluorescent nanovesicle as claimed in claim 1 does not undergo chemical degradation, neither aggregation thereby the fluorescence is stable at least over two months.

Moreover, the fluorescent nanovesicle according to the first aspect is designed to be able to interact with a water-soluble organic dye or non-water soluble organic dye, making the new fluorescent scaffold more versatile and suitable for using as a fluorescent probe independently of the solubility degree of the dye.

In a preferable embodiment of the organic dye of this invention, the solubility degree includes a water-soluble organic dye like as a water-soluble anionic dye, and a non-water soluble organic dye like as an amphiphilic organic dye or a fluorescent dye of a sterol derivative.

In another preferable embodiment, the organic dye interaction with hydrophobic and/or hydrophilic regions of the bilayer membrane includes a covalent binding, a hydrophobic binding or an electrostatic binding.

That is, the fluorescent nanovesicles according to the present invention comprise at least an organic dye, comprising the nanovesicle a bilayer membrane including a sterol molecule or derivatives thereof assembled with a quaternary ammonium surfactant molecule in which the at least an organic dye interacts with said bilayer membrane.

In a preferable embodiment, the nanovesicle can have an average size of from 25 to 500 nm as measured by dynamic light scattering and nanoparticle tracking analysis, still more preferable from 50 nm to 300 nm. In a still preferable embodiment, the nanovesicle can be substantially spherical in shape.

Geometrically, the quaternary ammonium surfactant molecule has a conical shape, whereas the sterol molecule has an inverted conical shape, and the assembly of the conical molecule and the inverted conical molecule forms a synthon structure, which has a quasi-cylindrical shape.

A pair of synthons can form a closed and concentric bilayer membrane having a hydrophobic region in the central zone of the bilayer membrane and two hydrophilic regions around said central zone, corresponding to the inner layer separating the small enclosed aqueous liquid compartment, the lumen, and the outer layer of the bilayer membrane. The surfactant molecules can be arranged into two-layered leaflets with the head groups in contact with the aqueous medium outside the nanovesicles, and the tails in the interior of the bilayer, whereas sterol molecules can arrange themselves, including the hydroxyl groups, in the hydrophobic region generated by the surfactant tails to avoid any contact with the aqueous medium of the inner compartment or of that of the outside the nanovesicles.

Therefore, the nanovesicles according to this invention are amphiphilic nanovesicles.

Specifically, the organic dye interaction with hydrophobic and/or hydrophilic regions of the bilayer membrane can be a covalent binding to the sterol molecule in the hydrophobic region, a hydrophobic binding at the hydrophilic and hydrophobic regions, or an electrostatic binding at the outer hydrophilic region of the bilayer membrane.

In a preferable embodiment, the fluorescent dye of a sterol derivative is covalently bonded to the sterol molecule in the hydrophobic region of the bilayer membrane.

In a preferable embodiment, the amphiphilic organic dye is linked at the hydrophilic and hydrophobic regions of the bilayer membrane by a hydrophobic bond, in which the hydrophobic part of the amphiphilic dye is in contact with the hydrophobic region of the membrane, while the polar part of the amphiphilic dye is in contact with the hydrophilic region of the membrane.

In a preferable embodiment, the water-soluble anionic dye is electrostatically bonded at the outer hydrophilic region of the bilayer membrane.

In a preferable embodiment, the stable fluorescent nanovesicle according to the first aspect of this invention comprises at least two organic dyes that interact simultaneously in the same nanovesicle, wherein the at least two dyes, with different optical spectra, are selected in such a way that the emission spectrum of a dye partly overlaps with the absorption spectrum of another dye and the dyes are at a separate distance typically lower than 10 nm, as needed to observe Forster resonant energy transfer (FRET). The FRET effect is disclosed, for example, by Terenziani et al., "Dipolar versus Octupolar Triphenylamine-Based Fluorescent Organic Nanoparticles as Brilliant One- and Two-Photon Emitters for (Bio)imaging" from Small 2011, 7, No. 22, 3219-3229.

Advantageously, the fluorescent nanovesicle can comprise at least two organic dyes whose conditions allow generate the FRET effect. Thus, the fluorescent nanovesicles can be used as fluorescence resonance energy transfer (FRET)-based scaffolds with different emission spectra, that can be excited by a single wavelength excitation source as needed for multicolour detection applications.

In a preferable embodiment, the at least two organic dyes can be two non-water soluble organic dyes, or can be two water-soluble organic dyes, wherein the non-water soluble organic dye and the water-soluble organic dye have the same definitions as above.

The fluorescent nanovesicles can be excited by both one-photon and multi-photon excitation. Advantageously, the fluorescent nanovesicles can be designed for in vivo and in vitro imaging application in human and animal tissues, for excitation with different laser sources, and reaching different depths. Additionally or alternatively, the stable fluorescent nanovesicle can have a bioactive compound and/or a targeting agent.

The fluorescent nanovesicles can be functionalised with large numbers and varieties of functional molecules, allowing their use as biosensors.

Thus, the fluorescent nanovesicles can be functionalised using a plurality of ligands. Suitable ligands are known in the art. In some embodiments, the ligands can be attached to the outer layer of the fluorescent nanovesicles, for example, by coordination bonds. Ligands can also be associated with the outer layer of the fluorescent nanovesicles via non-covalent bond. The ligands can comprise coordinating or bonding functional groups, such as thiol, amine, phosphine, CO, $N_2$, alkene, chloride, hydride, alkyl, and derivatives thereof, and combinations thereof.

It is well understood by a skilled person in the art that the various optional features disclosed herein can be combined with each other whenever technically possible without departing the scope of protection of the present invention.

In a further aspect, this invention provides a stable colloidal dispersion comprising a plurality of stable fluorescent nanovesicles according to the first aspect of the present invention.

Surprisingly, the stability of the fluorescent nanovesicles of this invention upon dilution is excellent. In a preferable embodiment, the term stable can refer to the stability of the average size of the fluorescent nanovesicles over time and/or upon dilution.

Advantageously, the colloidal dispersion comprising a plurality of stable fluorescent nanovesicles can maintain its stability in an aqueous medium even upon dilution. Particularly, the stability upon dilution of the colloidal dispersion comprising a plurality of fluorescent nanovesicles according to the first aspect of this invention is maintained even when compared with a different system such as micelles loaded with the same organic dye, at a dilution concentrations below the critical micellar concentration (CMC) required to form the micelles.

Therefore, the colloidal dispersion of this invention can be advantageously used in applications where the concentration of the fluorescent nanovesicles is diluted in use like when they are used under physiological conditions such as in body fluids.

Therefore, the fluorescent nanovesicles disclosed herein have potential applications in a number of fields such as imaging (e.g., biological imaging, fluorescence imaging, biomedical imaging), sensing (e.g., chemical sensing, biological sensing), theranostic and medical applications (e.g., imaging, therapy, diagnostics, photothermal therapy, combinations thereof).

In other words, also disclosed herein are devices comprising the fluorescent nanovesicles disclosed herein. Examples of devices include, but are not limited to optical devices (e.g., light emitting diodes), optoelectronic devices, bioanalytical devices, chemical sensors, biosensors, and combinations thereof.

As stated above, the nanovesicle can comprise a sterol molecule and a quaternary ammonium surfactant molecule. The nanovesicle can, for example, be formed by self-assembly of the sterol and the quaternary ammonium surfactant molecule. As used herein, a lipid is not a surfactant, such that the nanovesicles are not liposomes.

In some embodiments, the sterol molecule or a derivative thereof, and the quaternary ammonium surfactant molecule can be present in the nanovesicle in a molar ratio of from 10:1 to 1:5, preferably a molar ratio in the range of 2:1 to 1:2, still more preferably a molar ratio of 1:1.

In a preferable embodiment, the sterol can be cholesterol.

In a preferable embodiment, the quaternary ammonium surfactant can be cetyl trimethylammonium bromide (CTAB), tetradecyldimethylbenzylammonium chloride (MKC), cetrimide and benzalkonium chloride (BKC) or mixtures thereof.

In a preferable embodiment, the bilayer membrane of the nanovesicle comprises a cholesterol and a quaternary ammonium surfactant. In some examples, the bilayer membrane comprises cholesterol and cetyl trimethylammonium bromide (CTAB).

An additional object of the present invention is to provide a practical method capable of preparing the stable fluorescent nanovesicles according to the first object of the invention, whose method is suitable to be scalable at industrial scale.

To solve this problem, the present invention provides a method as claimed in the attached claims for preparing the stable fluorescent nanovesicles according to the first aspect of the present invention.

In a preferable embodiment, forming the nanovesicles can be based on a one-step scalable method using $CO_2$ expanded solvents called Depressurization of an Expanded Liquid Organic Solution-suspension (DELOS-SUSP).

The method for forming the fluorescent nanovesicles based on a one-step scalable method using $CO_2$ expanded

solvents, called Depressurization of an Expanded Liquid Organic Solution-suspension, comprises the following steps: a) preparation of an aqueous solution of the quaternary ammonium surfactant, b) dissolution of the sterol or derivatives thereof in an organic solvent expanded with a compressed fluid (CF), and c) vesicle synthesis by depressurization of the resulting solution in step b) on the solution resulting in step a), and it is characterized in that the method further comprises:

being the organic dye a water-soluble organic dye, further:

in step a), dissolving the water-soluble organic dye with the aqueous solution of the quaternary ammonium surfactant, and then proceed with the above steps b) to c); or in an alternative way, adding the water-soluble organic dye directly at the nanovesicles prepared in step c); and

being the organic dye a non-water soluble organic dye, further:

in step b), dissolving the non-water soluble organic dye with the organic solution expanded with the CF, and then, proceed with step c).

wherein the meaning of the water-soluble organic dye, the non-water soluble organic dye, the quaternary ammonium surfactant, the sterol or derivatives thereof, and the molar ratio of the quaternary ammonium surfactant molecule to sterol molecule or derivatives thereof are the same as described herein.

Non-liposomal nanovesicular structures can be formed, for example, using equimolar amounts of sterols, such as cholesterol (chol), and quaternary ammonium surfactants, such as CTAB. None of the individual components of a nanovesicle self-assemble to form vesicular structures, since in water quaternary ammonium surfactants form micelles and the insoluble sterol species form crystals, though in combination the components form amphiphilic bimolecular building blocks that assemble into closed bilayers. These nanovesicles do not tend to aggregate, and they keep their structure for periods as long as several years.

The obtained nanovesicular structures exhibit a high nanovesicle to nanovesicle homogeneity regarding size, lamellarity, and membrane supramolecular organization, which are all properties that can impact the use of such nanovesicles in medical or diagnosis imaging. Contrary to micellar structures formed exclusively with quaternary ammonium surfactants, the morphology of these nanovesicules is substantially unaffected upon increasing the temperature or by dilution, making them attractive candidates for use *in-vivo.* The nanovesicles are stable aqueous colloidal structures and they have antibacterial and anti-biofilm properties.

In a preferable embodiment, the organic solvent is selected from a monohydric alcohol a polyhydric alcohol, a ketone, ethylenediamine, acetonitrile, ethyl acetate and mixtures thereof; and the CF is selected from selected $CO_2$, ethane, propane, a hydrochlorofluorocarbon, and a hydrofluorocarbon.

Surprisingly, the interaction of the organic dye with the bilayer membrane does not disturb the stability of the nanovesicle, nor migration of the organic dye to the aqueous phase is detected.

There is not a specific threshold organic dye concentration. However, it is postulated that the threshold organic dye concentration is function of the maximum interaction, which is possible of the organic dye with the nanovesicle that at the same time does not affect the stability of the nanovesicles themselves. It is well understood by a skilled person in the art that this threshold concentration can vary depending on the specific organic dye and on the specific type of interaction. However, for all tested organic dyes fluorescent properties are stable for over two months, indicating that the organic dye was stable in this media, being the organic dye covalently bonded, hydrophobically bonded or electrostatically bonded to the bilayer membrane of the nanovesicle.

Advantageously, the method for forming the fluorescent nanovesicles according to this invention provides an adequate covalent binding, hydrophobic anchorage and electrostatic binding to the bilayer membrane of the nanovesicle. Advantageously, the method for forming the fluorescent nanovesicles according to this invention also provides chemical and physical stability of the nanovesicle containing the dye in aqueous media.

Therefore, the present invention also encompasses a fluorescent nanovesicle, which has been obtained by the forming method disclosed above.

Another aspect of this invention is the use of a stable fluorescent nanovesicle according to the first aspect of this invention as a fluorescent probe in an aqueous medium.

In a preferable embodiment, the fluorescent probe can be used for bioimaging and/or biodetection in an *in vitro* or *in vivo* aqueous media.

**Definitions**

According to the scope of the present invention, the term "aqueous medium" or "aqueous media" is intended to encompass/to mean a liquid system in which the main component is water, including a cell media (*in vitro*), and any physiological condition such as a body fluid (*in vivo*). The main component is water means that at least 50% is water.

In the present invention, the term "nanovesicles" refers to colloidal vesicles, which are between 25 nm and 5 $\mu$m in size and are formed by one bilayer of amphiphilic molecules that contain an aqueous phase in the small-enclosed liquid compartment. In particular, the nanovesicle comprises a bilayer membrane having hydrophobic and hydrophilic regions, in which the bilayer membrane comprises a sterol molecule and/or derivatives thereof assembled with a quaternary ammonium surfactant molecule.

In the invention, the term "quaternary ammonium surfactant" refers to those cationic surfactants with at least one positive charge in the molecule and also includes the combination of one or more cationic surfactants. Particularly, quaternary ammonium surfactants are quaternary ammonium salts in which at least one nitrogen substituent is a long chain. Compounds such as CTAB, cetrimide and BKC or their mixture are included. The cationic surfactants can be obtained from commercially available sources, with pharmaceutical and cosmetic qualities.

The term "derivatives" of sterol, in the present invention, includes derivatives of cholesterol and refers to molecules of the steroids family, generally obtained from the cholesterol precursor molecule and having lipophilic character.

The term "organic dye" refers to an organic molecule, which is capable to absorb light in the visible region, and possibly emits light in the same region upon light irradiation.

The term "fluorescence stability" in the present invention refers to the stability over time of the emission spectra of fluorescent nanoparticles in an aqueous medium.

The term "stable fluorescent nanovesicle" in the present invention refers to the above fluorescence stability and to the chemical and physical stability of the nanovesicles in an aqueous medium.

The term "organic solvent" which has been used in the method for forming the fluorescent nanovesicles according to this invention is a solvent selected from the group formed by monohydric alcohols, such as: ethanol, methanol, 1-propanol, 2-propanolol, 1-butanol, 1-hexanol, 1-octanol and trifluoroethanol; polyhydric alcohols, such as: propylene glycol, PEG 400 and 1,3-propanediol; ketones, such as acetone, methyl ethyl ketone and methyl isobutyl ketone; ethylenediamine, acetonitrile, ethyl acetate and mixtures thereof. In any case, whatever is the nature of the organic solvent, the lipid component has to be soluble in it and further, said solvent has to necessarily be miscible in CF and water. Moreover, the selected organic solvent must have relatively low toxicity.

The term "CF" which has been used in the method for forming the fluorescent nanovesicles according to this invention is a component selected between $CO_2$, ethane, propane, hydrochlorofluorocarbons (eg., CFC-22), and hydrofluorocarbons (eg., HFC-134A). Preferably, the CF in step b) is $CO_2$, considered an ecological solvent, because it is nontoxic, non-flammable, non-corrosive, is not harmful for the environment and moreover, is very abundant in nature.

**Brief Description of the Drawings**

**Figure 1** depicts size distribution of plain nanovesicles measured by Dynamic Light Scattering (DLS) and Nanoparticle Tracking Analysis (NTA).

**Figure 2** shows CryoTEM images of DiD-Nanovesicules (1.3 mol*10$^{-3}$) a) DELOS-SUSP, b) Ultrasonication, c) Thin Film Hydration, d) Incubation.

**Figure 3** depicts graphs of time evolution of DiD-nanovesicles (1.3 mol*10$^{-3}$) evaluated by absorption, emission (top) and size distribution over 2 months (bottom in combination with Table A).

**Figure 4** depicts graphs of absorption (top) and size evolution (bottom) of DiD-NPs over two weeks.

**Figure 5** depicts graphs of stability upon dilution of DiD-nanovesicles (1.3 mol*10$^{-3}$) (continuous lines) compared with DiD-CTAB micelles (disrupted lines).

**Figure 6** shows size distribution by NTA and CryoTEM images of DID-nanovesicles at increasing concentrations (from a→e); and the integration efficiency (EE) of DELOS-SUSP of DID-nanovesicles are included in Table B.

**Figure 7** depicts a graph of absorbtion of DiD-nanovesicles at different compositions.

**Figure 8** depicts a graph of absorption change upon irradiation of DID-nanovesicles (1.3 mol*10$^{-3}$) at different irradiation times.

**Figure 9** show bar charts of size (top), Z-potential (bottom) and CryoTEM images of Fluorescein sodium salt (FI)-nanovesicles at different compositions after one day of preparation and after 2 months.

**Figure 10** depict graphs of absorbance (top) and emission (bottom) of FI and FI-nanovesicles at different concentrations.

**Figure 11** depict graphs of fluorescence intensity change upon irradiation of FI-nanovesicles 0.5 mol*10$^{-3}$ (top) and normalized emission intensity changes upon irradiation (bottom) of samples with lowest FI concentration.

**Figure 12** show bar charts of size (top), Z-potential (bottom) and CryoTEM images of NBD6Chol-nanovesicles at different compositions after one day of preparation and after 1 month.

**Figure 13** depicts a graph of emission profiles along with the fluorescence and photodecomposition quantum yields of Chol6NBD-nanovesicles.

**Figure 14** is a graph comparative summary of DiD and FI, and a schematic representation of nanoparticles decorated with FI, in which emission of DiD-nanovesicles and DiD-NPs (top-left) and absorption spectra of DiD-nanovesicles prepared by different routes (right), and fluorescence intensity of FI and FI-nanovesicles under irradiation over time (bottom-right).

**Figure 15** depicts the FRET occurring between DiI (Donor) and DiD (acceptor), both dyes being loaded in the bilayer membrane of a nanovesicle (QS). Exciting the DiI, it is possible to collect the emission from DiD (top), and depicts the FRET (cascade FRET) occurring between DiI, DiD and DiR (bottom), which reveals that exciting the DiI it is possible to collect emission from DiD and DiR.

**Figure 16** depicts the one-(solid line) and two-photon (line+symbols) absorption spectra of DID and DID nanovesicles at two different loadings, 3*10$^{-3}$ (triangles) and 6.6*10$^{-3}$ (squares). This result is a proof of concept that the DiD and the DiD nanovesicles can emit under one-photon and multi-photon excitation.

## Detailed Description of the Invention

Hereinafter, the best mode for carrying out the present invention is described in detail.

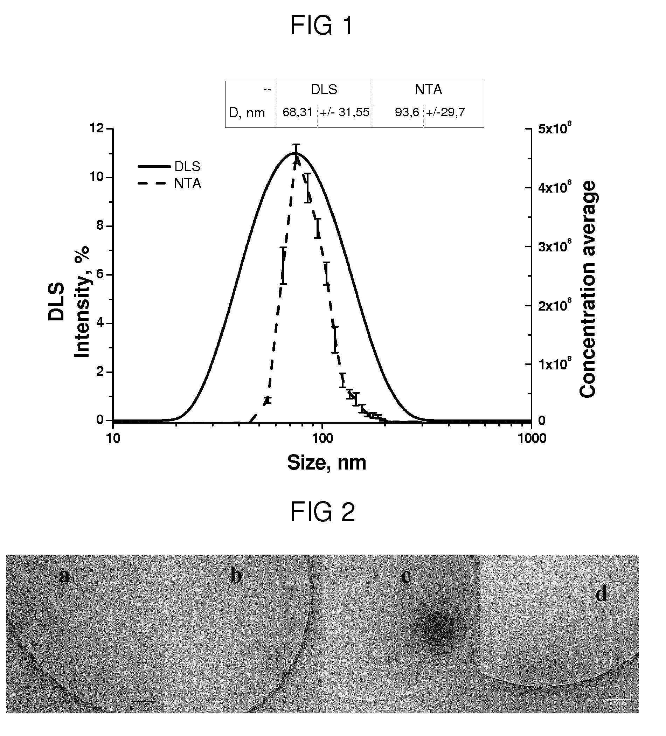

Here the authors of the present invention disclose a preferable embodiment of a fluorescent nanovesicle. In particular, three simple strategies will be described below to allow for the interaction of the organic dyes having different physicochemical and optical properties with the bilayer membrane of these nanovesicles, resulting in highly stable bright nanovesicles that show a great colloidal stability over time and a good photostability. The nanovesicles in this embodiment are formed by the self-assembly in water of CTAB and cholesterol in a 1:1 ratio (Figure 1) that create a supramolecular synthon with the proper shape and dimensions to self-assembly forming a spherical double-layer membrane with diameters of around 70 nm. Advantageously nanovesicles show a great colloidal stability during several years, as well as upon dilution and rising temperature, which is conferred by their strongly positively charged membrane. They can be prepared by the one-step method using compressed fluids (CFs), named Depressurization of Expanded Liquid Organic Solution-Suspension (DELOS-SUSP), which showed several advantages over conventional routes for the preparation of functionalized vesicles. Thanks to these properties nanovesicles resulted to be a versatile scaffold for the engineering of organic dyes-loaded vesicles using dyes with different physico-chemical properties, in terms of charge and solubility.

Three different interaction strategies depending on the nature and characteristics of the dyes:

- Lipophilic dyes with long alkyl chains can be "anchored" to the bilayer membrane by means of hydrophobic interactions which take place between the chains of the dyes and the hydrophobic compartment of the double-layer membrane. The dye used as a proof-of-concept for this embodiment was 1,1'-dioctadecyl-3,3,3',3'-tetramethyl-indodicarbocyanine perchlorate (DiD) a carbocyanine dye with two 18-carbons chains.

- Water-soluble and negatively charged dyes can be electrostatically adsorbed on the positively charged surface of nanovesicles. The dye used as a model for this strategy was fluorescein sodium salt, FI.

- Lastly, cholesterol of nanovesicle can be partially substituted by a cholesterol probe, as demonstrated herein by using NBD-6-Cholesterol, NBD6Chol leading to bilayer membrane nanovesicle with a covalently linked dye. In some examples, the concentration of the NBD-6-Cholesterol to the cholesterol molecule can be lower than 2%. Advantageously, a concentration of the cholesterol derivative lower than 2% does not disturb the morphology and sizes of the resulting fluorescent nanovesicles. In a preferable embodiment, the molar ratio of the cholesterol molecules, including NBD-6-Cholesterol, with respect to the quaternary ammonium surfactant molecules can range from 10:1 to 1:5, preferably in the range of 2:1 to 1:2, still more preferably 1:1.

Unlike fluorescein, DiD and NBD-6-Chol are not soluble in water. Hence, the innovation here reported is the use of the nanovesicles as robust and versatile scaffolds enabling nanostructuring in water a variety of dyes with different physico-chemical characteristics without losing their optical properties providing in some case an enhancement of their photostabilities.

Nanovesicles labelled with DiD (DiD-nanovesicles)

The interaction of the DiD with the nanovesicle was performed using the DELOS-SUSP methodology (Cano-Sarabia M et al. Langmuir 2008, 24, 2433-2437; Cabrera I et al. Nano Lett. 2013, 13, 3766-3774) and the membrane functionalization of resulting labeled nanovesicles, DiD-nanovesicles, compared with other methods generally used for loading vesicles, such as incubation (IC), ultrasonication (US) and thin film hydration (TFH). See below Example section for details on preparations.

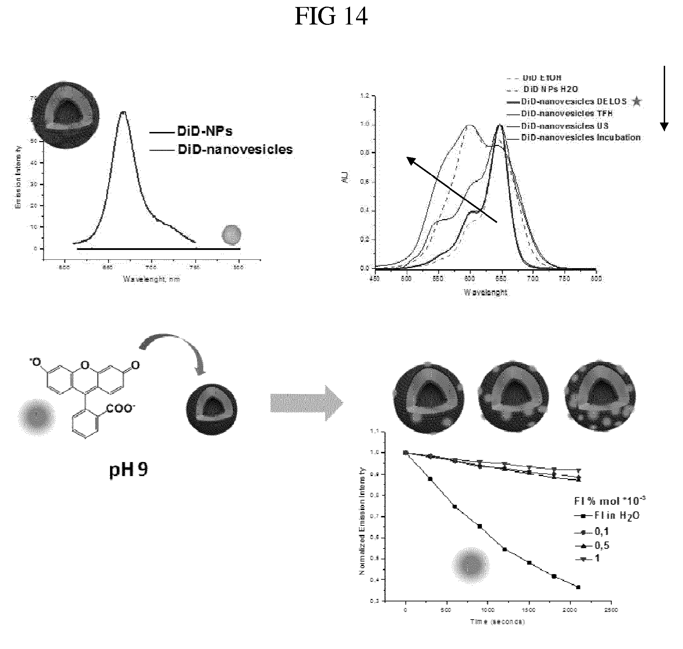

The normalized absorbances in the visible range of DiD-nanovesicles prepared by different routes are shown in Figure 14 for comparison along with the spectra of DiD in Ethanol (EtOH) and nanoparticles of pure DiD (DiD-NPs), prepared by DELOS method.

Cyanines dyes are well known for their tendency to self-aggregate at high concentrations, even in organic solvents. The band present at 550 nm, ascribed to the presence H-aggregates of DiD, is prominent in samples obtained by incubation and ultrasonication. The spectra of DiD-nanovesicles prepared by DELOS-SUSP and TFH are very similar to the spectrum of DiD in EtOH. This confirms that by DELOS-SUSP and TFH methods, isolated dye molecules are evenly distributed inside the membrane of nanovesicles. In any case, as shown in Figure 2, CryoTEM images of DiD-nanoparticles ($1.3 \ mol*10^{-3}$) prepared by different techniques. DiD-nanovesicles prepared by DELOS-SUSP are more homogenous in terms of lamellarity than DiD-nanovesicles by TFH. UV-vis spectra show that aggregates of DiD are forming depending on the preparation route followed. The image c) shows that by TFH it is impossible to obtain, without further purification steps, unilamellar nanovesicles with homogeneous size distribution.

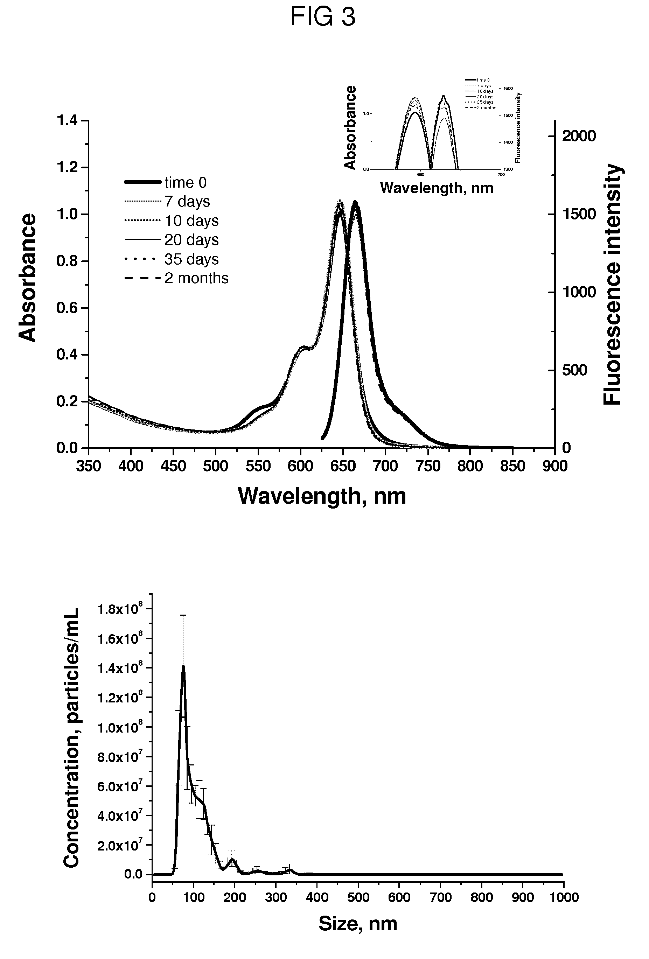

DiD-nanovesicles were found to be colloidally stable during months and no noticeable changes were found in the size distribution neither in absorbance/emission spectra over two months (Figure 3 and Table A).

Chemical stability of DiD-nanovesicles was studied by monitoring absorption and emission spectra of the samples over time. After an initial reorganization of the dye, the emission and absorbance are maintained over time. This proves the chemical and colloidal stability of the dye when inserted in nanovesicle's membrane, in fact it doesn't undergo chemical degradation neither aggregation over two months. See Figure 3.

Table A

| -- | After 1 month | | After 2 months | |
| --- | --- | --- | --- | --- |
| **Mean** | 104.8 | +/- 2.7 | 101.1 | +/- 3.6 |
| Mode | 76.8 | +/- 2.3 | 75.2 | +/- 3.3 |
| SD | 42.5 | +/- 6.3 | 50.9 | +/- 2.5 |
| D10 | 57.5 | +/-2.1 | 53.9 | +/- 1.6 |
| D50 | 83.5 | +/- 7.7 | 76.4 | +/- 3.2 |
| D90 | 149.5 | +/- 10.6 | 137.9 | +/- 6.9 |

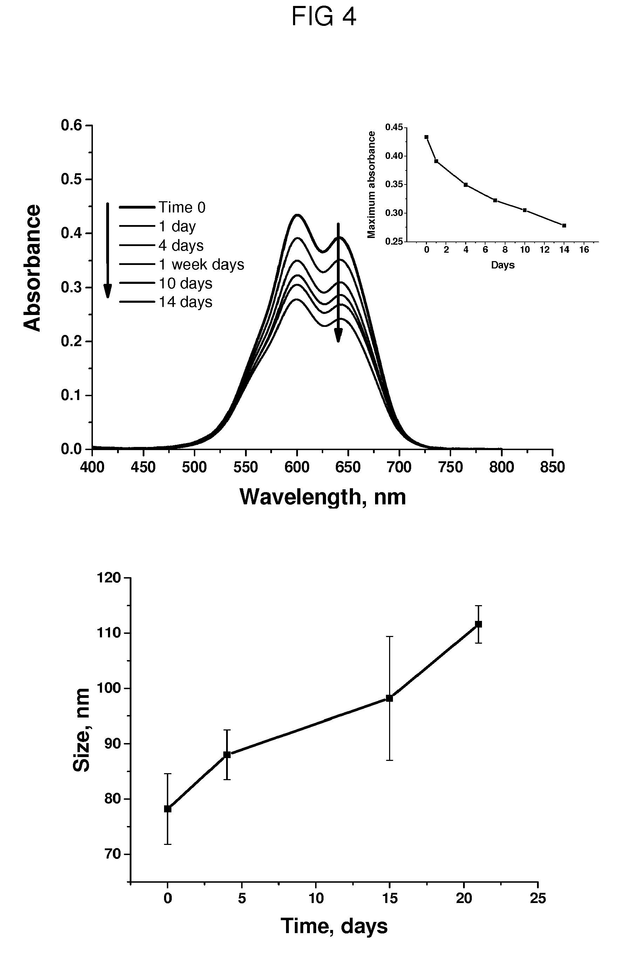

Moreover, the stability of DiD-nanovesicles was compared to that of DiD-NPs, as shown in Figure 4. DiD-NPs prepared by DELOS method were found to be unstable over time. The continuous decrease in absorption (top) is probably due to the aggregation occurring between the particles (bottom). DiD was found to precipitate after two months from the preparation of the nanoparticles.

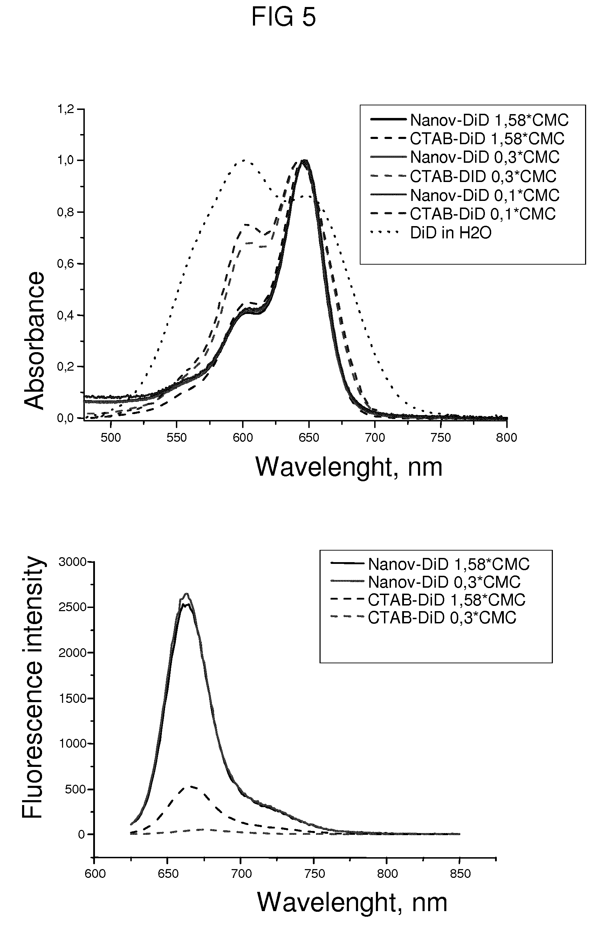

The advantages of the vesicular system DiD-nanovesicles over other formulations of DiD were analyzed by comparing DiD-nanovesicles with a sample of DiD-CTAB in which the CTAB micelles solubilize the DiD, which was obtained by DELOS-SUSP method but without cholesterol. The stability upon dilution (Figure 5) of DiD-nanovesicles (1.3 mol*$10^{-3}$) was studied by absorption (top) and emission (bottom) divided by absorption at excitation wavelength. The vesicular system was compared with a formulation of DiD-CTAB micelles. Absorption and emission at concentration above and below the Critical Micellar Concentration (CMC) of CTAB were compared. When diluting below CMC, normalized absorption of DiD-nanovesicles doesn't change while in DiD-CTAB some bands attributed to aggregation of DiD molecules start to appear. Moreover, DiD-nanovesicles do not lose brightness upon dilution while DiD-CTAB is almost not fluorescent at concentration lower than the CMC.

Advantageously, the stability upon dilution was excellent for DiD-nanovesicles, which maintains its brightness, while DiD-CTAB formulations do not fluoresce at concentrations below the critical micellar concentration (C<CMC) of CTAB. The effect of local concentration of DiD on the spectroscopic properties of DiD-nanovesicles was also studied.

Moreover, the fluorescence spectra (normalized by absorbance at excitation wavelength) of DiD-nanovesicles and DiD-NPs are compared, showing that the luminescence of the last formulation is quenched (Figure 14).

Integration efficiency (EE) of DELOS-SUSP was calculated by measuring the real concentration of DiD in nanovesicles and dividing by the nominal concentration (i.e. the theoretical concentration that the sample would have if all the DiD placed into the reactor would go inside nanovesicle's membrane).

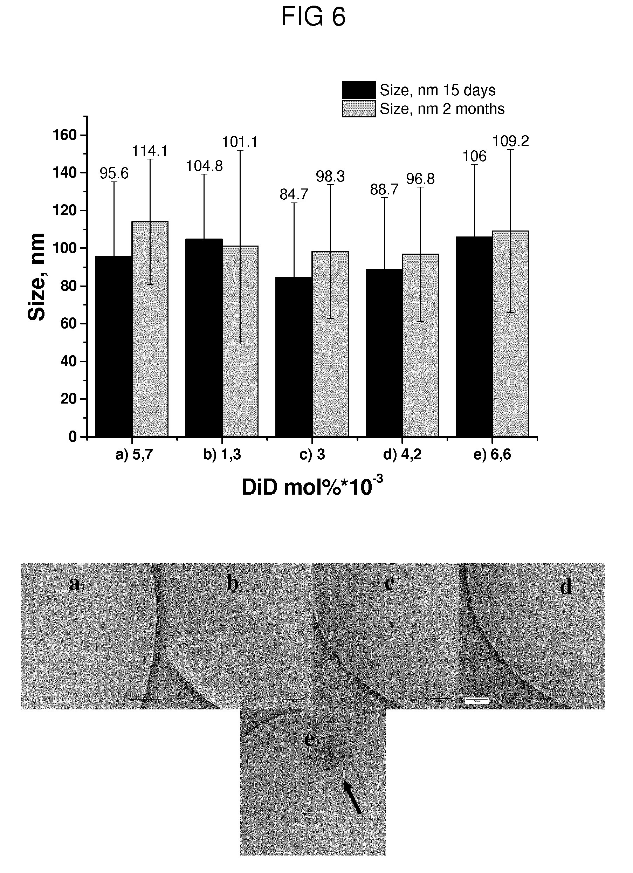

Five different samples with increasing fluorophore concentration were prepared by DELOS-SUSP, which all showed a high integration efficiency (Figure 6).

Table B

| DiD mol *$10^{-3}$ | Integration Efficiency of DELOS-SUSP |
| --- | --- |
| 0.57 | 0.8 |
| 1.3 | 0.9 |
| 3 | 0.84 |
| 4.2 | 0.79 |
| 6.6 | 0.92 |

Size distribution of said samples (Figure 6, top) shows that the increased concentration does not appreciably affect the size distribution and stability is confirmed over two months for all the samples. CryoTEM images (Figure 6, bottom) show good homogeneity. The black arrow in Fig 6e signs the presence of some other supramolecular structure in the suspension. Size distribution of the samples can be measured by Nanoparticle Tracking Analysis (NTA), equipped with a blue laser (488 nm), where DiD molecules do not absorb. CryoTEM images show that there is almost no effect of the dye on the morphology neither on lamellarity of the vesicles. Only in the sample with the highest DiD concentration some structures not attributable to nanovesicles appears, probably due to different supramolecular organization of DiD molecules.

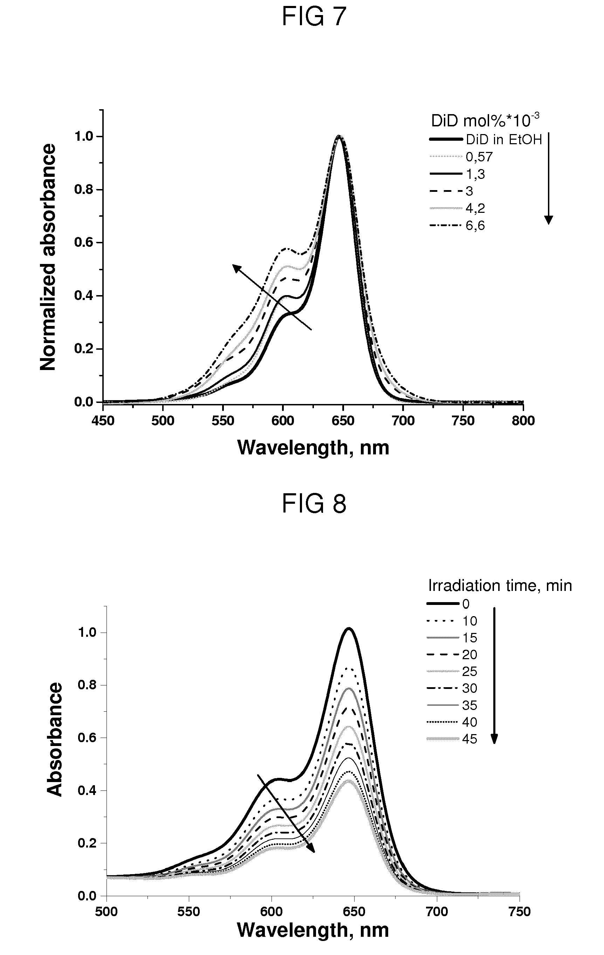

UV-vis normalized absorption spectra (Figure 7) show that as more dye is loaded in the bilayer membrane of nanovesicles more aggregates are formed. Normalized absorption spectra of DiD-nanovesicles at different compositions are compared to that of DiD in EtOH. As long as DiD concentration increase, a deformation of the band-shape with increase absorbance on the blue-side is observed, possibly due to the presence of some dimers and aggregates on the nanovesicles membrane.

Likely these aggregates are mainly present on the bilayer membrane of the nanovesicles itself, considering that CryoTEM images do not evidence the presence of other supramolecular structures, except in a few cases such the one previously mentioned. Fluorescence quantum yield (QY) of DiD-nanovesicles, see Table 1 below, decreases at high DiD concentrations. QY of DiD and Nanovesicles-DiD was measured exciting at 631 nm. Cresyl Violet in Methanol was used as standard. Photodecomposition QY was also measured.

The decrease of QY is ascribed to the proximity effect of the dye molecules and/or polarity effect of the solvent considering that it is likely that part of the dye stays in contact with water. Table 1 also shows a different composition of the nanovesicle, particularly PEG-Chol/Chol/CTAB, and Chol/MKC at different molar ratios, as well as different Cyanines dyes and more than one dye at the same nanovesicle to obtain fluorescent nanovesicles showing the FRET-effect.

Table 1

| Membrane anchoring | | | | |
|---|---|---|---|---|
| Nanovesicles composition and molar ratio | Probe (dye) | Probe concentration $*10^{-3}$ (mol/mol$_{tot}$) | QY $\Phi$(%) | Observations |
| Chol/CTAB 1/1 | DiD | 0.57 | 23 | Uptake in epithelial cells and feasible for STORM imaging |
| / | / | 1.3 | 19 | |
| / | / | 3 | 11 | |
| / | / | 4.2 | 10 | |
| / | / | 6.6 | 7 | |
| / | DiI | 0.57 | 18 | STORM imaging |
| / | / | 3 | 13 | |
| / | / | 6.6 | 10 | |
| PEG-Chol/Chol/CTAB 1/25/26 | / | 0.57 | 20 | STORM imaging |
| PEG-Chol/Chol/CTAB 1/6/7 | / | 0.57 | 20 | STORM imaging |
| Chol/CTAB 1/1 | DiR | 0.57 | 3 | |
| / | / | 6.6 | 1 ca. | |
| / | DiI+DiD (FRET sample) | 0.57 (DiI) - 0.57 (DiD) | (FRET ratio≈35-40%) | |
| / | / | 6.6 (DiI) - 6.6 (DiD) | (FRET ratio≈55-60%) | |
| / | DiI+DiD+DiR (cascade FRET) | 0.57 (DiI) - 0.57 (DiD)-0.57 (DiR) | | |
| / | / | 6.6 (DiI) - 6.6 (DiD) - 6.6 (DiR) | | |
| Chol/MKC 1/2 | DiR | 0.57 | | Injected in mousses (biodistribution study) |
| • DiI (1,1'-dioctadecyl-3,3,3',3'-tetramethyl-indocarbocyanine perchlorate) • DiR (1,1'-dioctadecyl-3,3,3',3'-tetramethylindotricarbocyanine iodide) | | | | |

Photostabilities of DiD in EtOH and of DiD-nanovesicles were analyzed by monitoring the evolution over time of absorbance of the samples under continuous laser irradiation. The data are shown in Table below and are also represented in Figure 8.

Additional column in Table 1 for rows of DiD

| Probe | Photodecomposition QY$*10^{-5}$ |
|---|---|
| DiD EtOH | <0,008 |
| DiD mol $*10^{-3}$ (mol/moltot) | Photodecomposition QY$*10^{-5}$ |

(continued)

| Probe | Photodecomposition QY*10$^{-5}$ |
|-------|------------------------------------|
| 0.57 | 1.3±0.4 |
| 1.3 | 1.4±0.2 |
| 3 | 1.9±0.3 |
| 4.2 | 1.9±0.4 |
| 6.6 | 1.8±0.4 |

The quantum yields of photoreaction were calculated following the method proposed by Belfield et al. "Photophysical and photochemical properties of 5,7-di-methoxycoumarin under one- and two-photon excitation". J. Phys. Org. Chem. 16, 69-78 (2003).

No relevant effect of DiD concentration was noticed on photostability of DiD-nanovesicles.

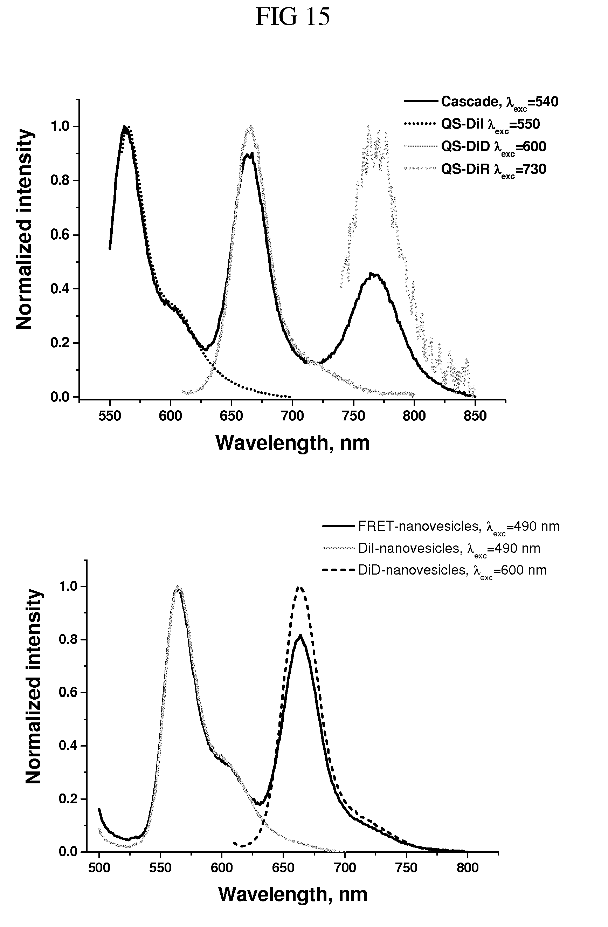

The resonance energy transfer between DiI and DiD (top) and cascade resonance energy transfer (FRET effect) between DiI, DiD and DiR is shown in Figure 15, top and bottom. Said Figures show that by selectively exciting the DiI (donor) it is possible to collect emissions of the acceptors, DiD (top) and DiD-DiR (bottom). Thus, the fluorescent nanovesicles can be used as fluorescence resonance energy transfer (FRET)-based scaffolds with different emission spectra, that can be excited by a single wavelength excitation source resulting in a tunable emission wavelengths.

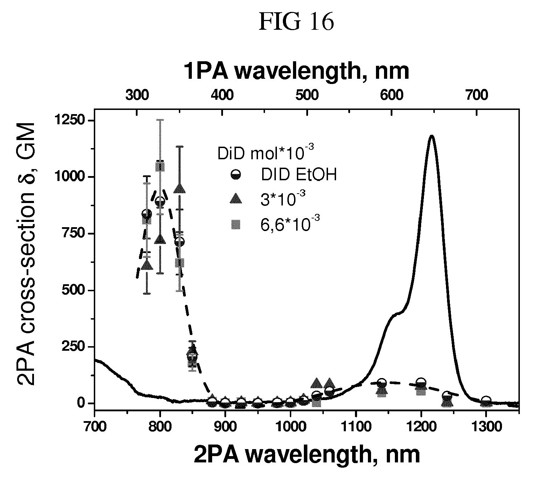

Moreover, the fluorescent nanovesicles can fluoresce both upon one-photon and multi-photon excitation and therefore, they can be designed to imaging at different deeps in a human or animal tissue (Figure 16).

Nanovesicles decorated with Fluorescein (Fl-nanovesicles)

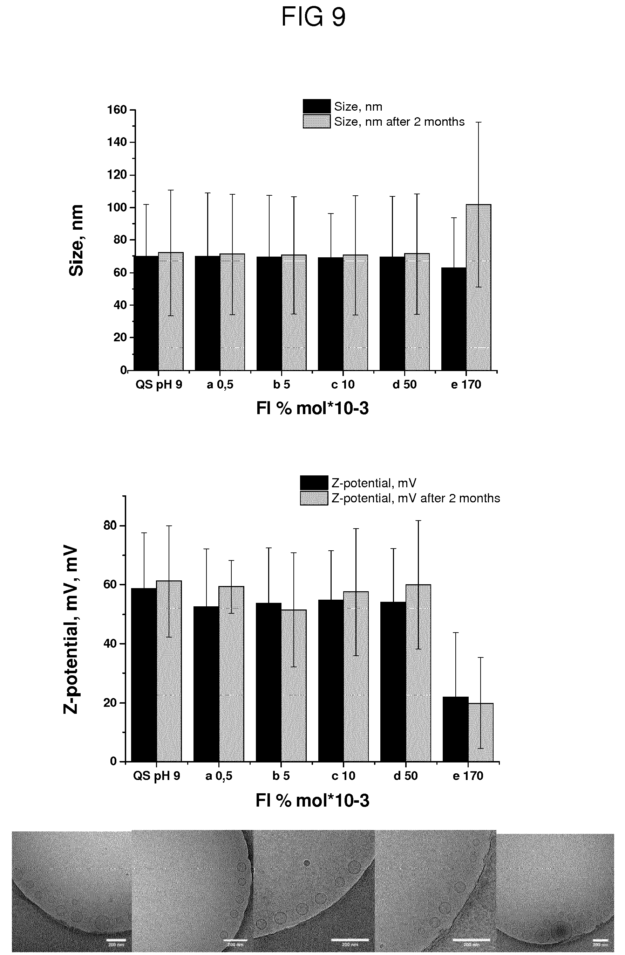

The ability of nanovesicles containing cationic or anionic (or even catanionic) surfactants to sequestrate charged solutes by electrostatic interactions has been already shown. We used this strategy to nanostructure on the surface of nanovesicles the anionic fluorescein dye in a basic water taking advantage of the cationic head of CTAB molecules. Size and Z-potential of resulting Fl-nanovesicles with different concentrations of Fl were measured by DLS (Figure 9, bar charts), along with CryoTEM images of the samples (Figure 9, bottom).

No relevant variations on nanovesicle size have been noticed for effect of Fluorescein addition to Nanovesicles. Z-potential decreases at higher concentrations of Fl, probably as shielding effect of Fl over the positive membrane of Nanovesicles. CryoTEM images as well don't show any relevant changes with higher concentration of Fl.

Samples were found to be stable over two months. No differences in sizes were noticed, while Z-potential decreases of around 63% for the sample with the highest concentration of Fl, due to the shielding effect of di-anionic fluorescein on the positive charge of CTAB. The electrostatic interaction between Fl and CTAB on nanovesicle's surface is reversible and accordingly it was possible to remove the Fl from nanovesicle surface by diafiltration. Free Fl in solution was detected in the samples with high concentration of dye.

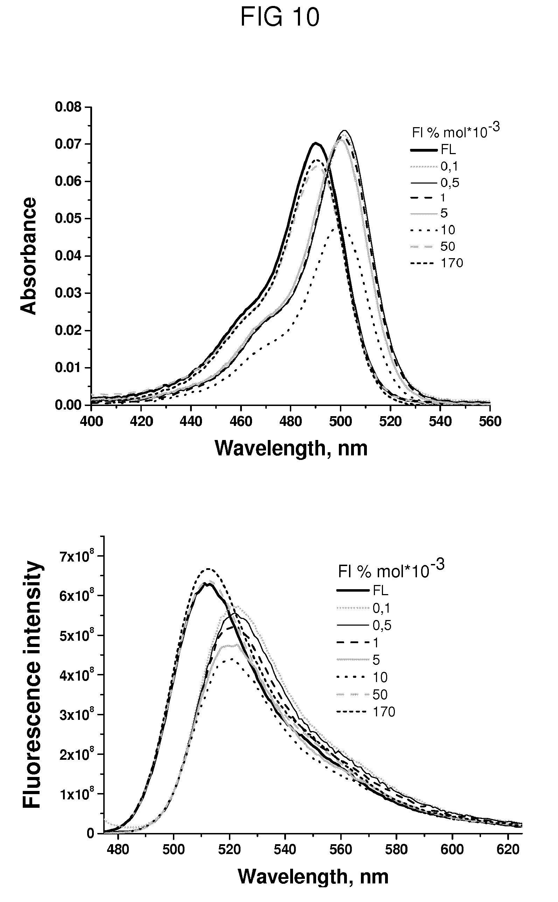

Absorbance and emission spectra of Fl-nanovesicles are shown in Figure 10. The electrostatic interaction of Fl with CTAB produces a red-shift in absorption (top) and emission (bottom) spectra. The Stoke's shift (in cm$^{-1}$) increases at higher fluorophore content due to the presence of free Fl. Increasing the concentration, the absorbance and emission intensities decrease as well. QY was calculated exciting the sample at 480 nm. The photodecomposition QY was measured only for the samples in which no free Fl was detected by purification. Optical properties of Fl-nanovesicles at increasing fluorophore concentration are listed in Table 2 below.

Table 2

| Electrostatic interaction | | | | |
|---------------------------|------------|------------------------------------------|---------|------------|
| Nanovesicles composition | Probe (dye) | Probe concentration *10$^{-3}$ (mol/mol$_{tot}$) | QY Φ (%) | PQY*10$^{-5}$ |
| Chol/CTAB - 1/1 | Fluorescein | 0.1 | 93 | 2.6±1.0 |
| / | / | 0.5 | 89 | 2.5±0.5 |
| / | / | 1 | 78 | 1.4±0.616 |
| / | / | 5 | 73 | |

(continued)

| Electrostatic interaction | | | | |
|---|---|---|---|---|
| Nanovesicles composition | Probe (dye) | Probe concentration $*10^{-3}$ (mol/mol$_{tot}$) | QY $\Phi$ (%) | PQY$*10^{-5}$ |
| / | / | 10 | 67 | |

As effect of high concentration of Fl over nanovesicle membrane a self-quenching phenomenon occurs, as shown by the decrease of fluorescence QY. At higher concentration, the contribution of free Fl gets higher as shown by the increase in QY coupled with an increase in the Stoke's shift.

Photostability of Fl-nanovesicles was studied as already shown for DID-nanovesicles, but in this study the emission of the dye upon irradiation was monitored. The evolution of the fluorescence at different irradiation times is shown in (Figure 14), while the photodecomposition QYs of the samples are shown in Table 2 above.

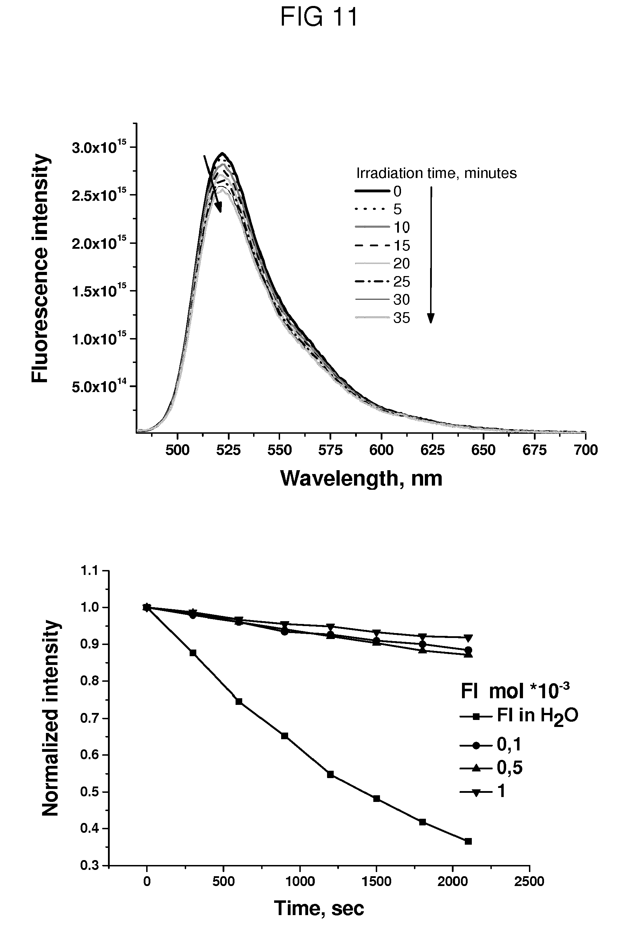

Only the samples with lower Fl contents were monitored in order to exclude any possible contribution of free Fl in water to the observed emission. Emission intensity of Fl decreases more than 60% after 40 minutes of irradiation, while decreases less than 15% when it is attached over nanovesicle's surface, showing how the surrounding of the dye molecule affects its rate of degradation (Figure 11). The electrostatic interaction with the nanovesicle's membrane probably hinders the contact with oxygen, limiting the bleaching of the dye, making therefore, fluorescein more photostable (up to three times) when attached to nanovesicles.

Nanovesicles labeled with NBD-6-Cholesterol (NBD6Chol-nanovesicles)

Cholesterol was partially substituted by NBD-6-Cholesterol for the preparation by DELOS-SUSP method of nanovesicles with this dye covalently attached to their membranes. Five samples were prepared with different NBD-6-Chol/Chol ratios by DELOS-SUSP shown in the Table 3 below.

Table 3

| Covalent binding | | | |
|---|---|---|---|
| Nanovesicles composition | Probe (dye) | Probe concentration $*10^{-3}$ (mol/mol$_{tot}$) | QY $\Phi$ (%) |
| Chol/CTAB 1/1 | NBD-6-Cholesterol | 0.46 | 8 |
| / | / | 1.3 | 4 |
| / | / | 2.2 | 4 |
| / | / | 5.2 | 2 |
| / | / | 6.4 | 3 |

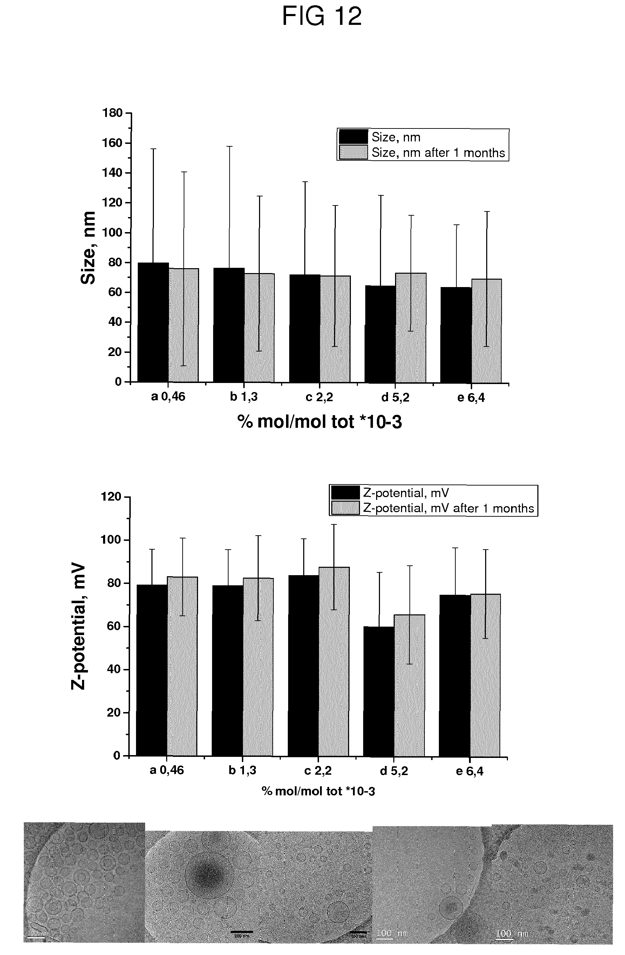

The ratio between NBD-6-Chol and Chol was held lower than 2% in order to have minor effects on the morphology and sizes of the resulting NBD6Chol-nanovesicles. Size and Z-potential (measured by DLS) of the samples over 1 month (Figure 12) showed a constant and homogenous size distribution revealing they are stable with high positive Z-potentials ($\approx$ 80mV). Likewise CryoTEM micrographs (Figure 12) showed homogeneous structures confirming that the partial substitution of cholesterol does not affect the morphology of the vesicles.

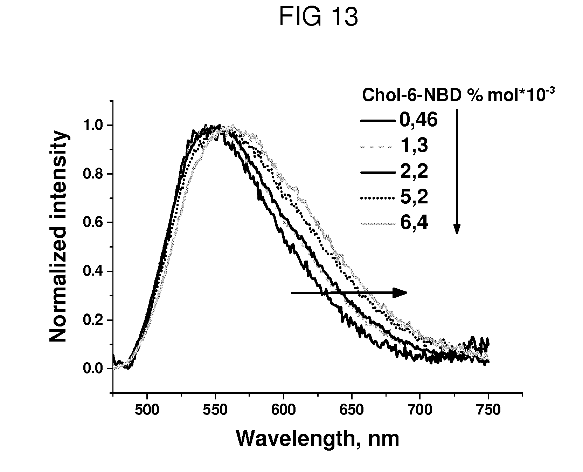

Therefore, no relevant variations have been noticed on distribution of size and Z-potential and CryoTEM images show homogeneous and unilamellar vesicles at all the concentration assayed. The increased concentration of NBD-6-Chol, NBD6Chol-nanovesicles reduces the QYs of the samples as can be seen in Table 3 above and Figure 13, probably due to aggregations of the dyes on nanovesicle's membrane or the proximity of the NBD groups on nanovesicle's surface. This assumption is further supported by the broadening and the red-shift of the emission spectra (Figure 13).

Although the scope of the present invention is not restricted to a selected organic dye, it is preferable that the organic dye be a water-soluble anionic dye, an amphiphilic organic dye or a fluorescent dye of a sterol derivative.

**Examples**

Hereinafter, the present invention is described in more detail and specifically with reference to the Examples and Figures, which however are not intended to limit the present invention.

## Materials

5-Cholesten-3β-ol (Chol, purity 95%) was purchased from Panreac (Barcelona, Spain). Hexadecyltrimethylammonium bromide (CTAB, BioUltra for molecular biology ≥99.0%) was purchased from Sigma-Aldrich. Fluorescein sodium salt (FL, 98.5-100.5% of purity) was obtained from Fluka. Sodium hydroxide (NaOH, ≥98,0%) was obtained from Panreac. 1,1'-dioctadecyl-3,3,3',3'-tetramethyl-indodicarbocyanine perchlorate (DiD oil) was purchased from Life Technologies (Carlsbad, USA). NBD-6-Cholesterol was supplied by Avanti Polar Lipids (Alabaster, AL, USA). Milli-Q water was used for all the samples preparation (Millipore Ibérica, Madrid, Spain). Ethanol (Teknocroma Sant Cugat del Vallès, Spain) was purchased with high. Carbon dioxide (99,9% purity) was purchased by Carburos Metálicos S.A. (Barcelona, Spain). All the chemicals were used without further purification.

## Preparation of fluorescent nanovesicles by DELOS-SUSP

The formation of the fluorescent nanovesicles is based on the one-step DELOS-SUSP method. DELOS-SUSP (*depressurization of an expanded liquid organic solution-suspension*), a compressed fluids (CFs) based method, was used for the preparation of nanovesicles (Cano-Sarabia M et al. Langmuir 2008, 24, 2433-2437; Cabrera I et al. Nano Lett. 2013, 13, 3766-3774).

Briefly, 111 mgr of Cholesterol was first dissolved in 4.2 mL of EtOH at working temperature $T_w$ ($T_w$=308 K) along with the hydrophobic dye (DiD or NBD-6-Chol). The solution was then added to a high pressure vessel (V=11.8 mL) at atmospheric pressure and $T_w$. After 20 minutes of equilibration the vessel was pressurized with $CO_2$ at the working pressure $P_w$ ($P_w$=10MPa) in order to have an expanded liquid ethanol solution with a molar fraction of $CO_2$ of $X_{CO2}$=0.63. The reactor was kept at the working condition for one hour, in order to homogenize the system. The organic solution was then depressurized over 35mL of water, where 100 mgr of CTAB had been previously dissolved. $N_2$ at 10MPa was added to the vessel during the depressurization in order to maintain constant Pw inside it. The vessel is equipped with a gas filter, in order to prevent any not solute compound to come out from the reactor during depressurization. With this one-step method, nanovesicles comprising a bilayer membrane with homogeneous size can be prepared. Furthermore it allows the straightforward loading of nanovesicles with hydrophobic compounds, such as DiD and NBD-6-Cholesterol, yielding the labeled nanovesicles: DiD-nanovesicles and NBD6Cholnanovesicles.

Five different samples of DiD-nanovesicles were prepared with the following molar ratios DiD/(Chol+CTAB): 0.57-1.3-3-4.2-6.6 mol*$10^{-3}$.

5 different samples of NBD6Chol-nanovesicles were prepared with the following molar ratios NBD-6-Chol/(Chol+CTAB): 0.46, 1.3, 2.2, 5.2, 6.4 mol*$10^{-3}$. In this case cholesterol was partially substituted by the NBD-6-Cholesterol with the cited ratios.

DiD Nanoparticles (DiD-NPs) were prepared by DELOS method. Briefly, DiD was dissolved in EtOH (without cholesterol) and placed inside the high pressure vessel. The same steps of DELOS-SUSP for nanovesicles preparation were followed, but depressurization was performed over MilliQ water (without CTAB).

CTAB micelles loaded with DiD (CTAB-DiD) were prepared by the same route, but depressurization was performed over water with CTAB.

Fl-nanovesicles samples were prepared by adding few uL of 0.1 mM Fl solution to plain nanovesicle at a pH = 9 (pH was modified by adding few drops of 0.01 M NaOH solution to preformed nanovesicle) in order to obtain 1 uM of Fl. Samples were gently shacked in an incubator at room temperature for 30 minutes. pH was modified up to 9 in order to have Fl as dianion. Different samples of Fl- nanovesicles were prepared with the following molar ratios Fl/(Chol+CTAB): 0.1-0.5-1-2.5-5-10-50-170 mol*$10^{-3}$.

## Preparation of DiD-nanovesicles by other methods

The ability of DELOS-SUSP for the straightforward integration of hydrophobic dyes, such as DiD, was compared with other routes for cholesterol-rich vesicles preparations, such as Ultrasonication (US) and Thin Film Hydration (TFH). The loading capability was as well compared with Incubation of preformed plain nanovesicles with DiD.

### *Thin Film Hydration (TFH):*

Proper quantities of membrane components, with same concentrations of DiD-nanovesicles prepared by DELOS-SUSP, were initially dissolved in chloroform. The solvent was then rotevaporated to remove the solvent and form a thin film. The film was further dried by placing it under vacuum for 4 hours. Once dried, the film was hydrated at room temperature over night using water+10%vol of EtOH. With a further step, the obtained nanovesicles were downsized by ultrasonication for 10 minutes.

*Ultrasonication (US):*

Proper quantities of Cholesterol and CTAB as shown for DELOS-SUSP and TFH were mixed in a vial. MilliQ water and a solution of DiD in Ethanol (in volume ratio 9:1) were then added to the solids immediately before placing the US tip inside the vial. The sample was sonicated for 8 minutes. No further steps were required.

*Incubation (US):*

Few uL of a concentrated solution of DiD in EtOH were added to preformed nanovesicles and incubated for 24 hours. During this incubation time the UV-vis absorption spectra of the sample was monitored. The sample was unstable over time.

Purification

All samples were purified by diafiltration by using KrosFlo Research II*i* TFF System (SpectrumLabs, USA) equipped with mPES MicroKros filter column (100 kDa MWCO) in order to remove ethanol and excess of CTAB.
FI-nanovesicles samples were not purified after addition of FI, because release of FI molecules was noticed during diafiltration. The presence of free dye was monitored by centrifugal filters (Centricon, Merck Millipore) with 100kDa cut off. After equilibrating three times with MilliQ water, 5 mL of samples were loaded into the Centricon and centrifuged. Presence of non-attached FI molecules was verified for samples with ratios CTAB/FI lower than 50.
The real concentration of DiD and Chol-6-NBD in nanovesicles was determined by adding a known volume of ethanol to the samples (volume ratio ethanol/nanovesicle sample in at least 10:1). The EtOH causes the rupture of the vesicles (verified by Nanoparticle Tracking Analysis NTA) and the dissolution of the dyes. The maximum absorption was then measured and divided by the calculated extinction coefficient of the dye in ethanol

$$(\varepsilon_i DiD^{\mathbf{T}}(MAX\ Abs) = [\![ 246500\ M ]\!]^{\mathbf{T}}(-1)\ [\![ cm ]\!]^{\dagger}(-1);\ \varepsilon_i(NBD-6-Chol)^{\mathbf{T}}(MAX\ Abs) = 22500\ M^{\dagger}(-1)\ [\![ cm ]\!]^{\mathbf{T}}(-1))$$

No effect of cholesterol and CTAB on shape and intensity of absorption bands of DiD and NBD-6-Chol in ethanol was detected.

Characterization of dye-labelled nanovesicles

Size and morphology

*Dynamic Light Scattering (DLS):*

Size, Polidispersity Index (PdI) and Z-potential of plain nanovesicles, FI-nanovesicles and NBD6Chol-nanovesicles were studied using Dynamic Light Scattering (Malvern Zetasizer Nano ZS, Malvern Instruments, UK) with non-invasive backscatter optics, equipped with He-Ne laser at 633 nm. All the values reported were the average of 3 consecutive measurements of the same samples at 25°C.

*Nanoparticle tracking analysis (NTA):*

Mean size and size distribution of DiD-nanovesicles were analyzed by NTA using a Nanosight NS300 equipped with a laser at 488 nm. It was not possible to study DID-nanovesicles by DLS due to the relevant absorbance of this sample at 633 nm. The sample is placed into a chamber and a laser beam is passed through it. Light scattered by the particles can be visualized by a 20X magnification microscope equipped with a sCMOS camera. The camera captures a video of the particles moving under Brownian motion. The diffusivity of the particles is then calculated by the software and the size calculated by using Stokes-Einstein equation. Samples were diluted 10000 times to fit the concentration range suggested by manufacturer. The values reported are average of results from five videos of each sample.

*Cryo-TEM images:*

Cryogenic transmission electronic microscopy images (Cryo-TEM) were acquired with a JEOL JEM microscope (JEOL, Tokyo, Japan) operating at 120kV. The sample was placed in a copper grid coated with a perforated polymer film and then plunged into liquid ethane to freeze it. Then it was placed into the microscope for the analysis.

Spectroscopic Measurements

UV-vis absorption spectra were recorded using a Lambda 650 (Perkin Elmer) spectrophotometer. Steady-state fluorescence and excitation spectra were collected with a Fluoromax-3 (Horiba Jobin Yvon) spectrofluorimeter for dilute solutions (maximum absorbance <0.1) in spectroscopic grade ethanol, methanol and MilliQ water. Fluorescence quantum yields were determined using comparative method with fluorescein in 0.1 M NaOH (QY, $\Phi$: 92%)and cresyl violet in methanol (QY, $\Phi$: 56%)as reference. Fluorescence decay curves were measured using time correlated-single photon counting method.

Sample photostability under continuous-wave excitation was determined by measuring the photochemical decomposition quantum yield. The photochemical decomposition quantum yield $\Phi_{ph}$ is defined as the ratio of number of bleached molecules $N_{mol}$ per number of absorbed photons $N_{phot}$:

$$\Phi_{ph} = N_{mol} / N_{phot}$$

Quantum yields of photochemical decomposition were measured in ethanol (for DID) and water (FI, NDB and all nanovesicle samples) using absorption and fluorescence methods, where the number of bleached molecules is determined by a kinetic decrease in absorbance or fluorescence intensity under the assumption that fluorescence and absorbance of the photoproducts are negligible in the spectral region of dye fluorescence and main absorption band. Fluorescence method was used to estimate the photostability of dilute samples (with maximum absorbance < 0.1), while photochemical decomposition quantum yields of concentrated solutions (with maximum absorbance $\approx$ 1) were determined by absorption method. The output of a Kr$^+$ laser (at 476.2 nm and 482.5 nm) and a diode laser (660 nm) was used for excitation of FI, NBD-6-Cholesterol and DID samples respectively. The entire volume of a sample was illuminated simultaneously in order to exclude the influence of diffusion on photochemical process.

It is a matter of course that the features mentioned above and those explained below can be used in other combinations in addition to those described, on in isolation, without departing from the scope of the invention.

**Claims**

1. A stable fluorescent nanovesicle comprising a bilayer membrane having hydrophobic and hydrophilic regions and at least an organic dye that interacts with hydrophobic and/or hydrophilic regions of the bilayer membrane, wherein the bilayer membrane comprises a sterol molecule or derivatives thereof assembled with a quaternary ammonium surfactant molecule, and wherein the fluorescent nanovesicle has a fluorescence stability for at least two months in an aqueous media.

2. The stable fluorescent nanovesicle according to claim 1, wherein the organic dye is selected from a water-soluble organic dye and a non-water soluble organic dye.

3. The stable fluorescent nanovesicle according to claim 2, wherein the water-soluble organic dye is a water-soluble anionic dye, and the non-water soluble organic dye is selected from an amphiphilic organic dye or a fluorescent dye of a sterol derivative.

4. The stable fluorescent nanovesicle according to any one of previous claims, wherein the organic dye interaction is selected from a covalent binding, a hydrophobic binding or an electrostatic binding.

5. The stable fluorescent nanovesicle according to any one of previous claims, wherein the fluorescent nanovesicle is chemically and physically stable in aqueous media.

6. The stable fluorescent nanovesicle according to any one of previous claims, wherein the quaternary ammonium surfactant is selected from CTAB, MKC, cetrimide and BKC or mixtures thereof, and wherein the sterol is cholesterol.

7. The stable fluorescent nanovesicle according to any one of previous claims, wherein the nanovesicles have a molar ratio of the quaternary ammonium surfactant to sterol or derivatives thereof in the range of 10:1 to 1:5, preferably a molar ratio of 1:1.

8. The stable fluorescent nanovesicle according to any one of previous claims comprising at least two organic dyes that interact simultaneously in the same nanovesicle, wherein the at least two dyes, with different optical spectra, are selected in such a way that the emission spectrum of a dye partly overlaps with the absorption spectrum of another dye and the dyes are at a separate distance typically lower than 10 nm.

9. The stable fluorescent nanovesicle according to claim 8, wherein the at least the organic dyes are non-water soluble organic dyes, or water-soluble organic dyes, wherein the non-water soluble organic dye and the water-soluble organic dye have the same definitions as above.

10. The stable fluorescent nanovesicle according to any one of previous claims, wherein the fluorescent nanovesicle further comprises a bioactive compound and/or a targeting agent.

11. A method for preparing a stable fluorescent nanovesicle comprising at least an organic dye according to any one of claims 1 to 10, the method comprising the following steps: a) preparation of an aqueous solution of a quaternary ammonium surfactant, b) dissolution of a sterol or derivatives thereof in an organic solvent expanded with a compressed fluid (CF), and c) vesicle synthesis by depressurization of the resulting solution in step b) on the solution resulting in step a),
**characterized in that** the method further comprises:

   being the organic dye a water-soluble organic dye, further:

      in step a), dissolving the water-soluble organic dye with the aqueous solution of the quaternary ammonium surfactant, and then proceed with the above steps b) to c); or in an alternative way, adding the water-soluble organic dye directly at the nanovesicles prepared in step c);
      and

   being the organic dye a non-water soluble organic dye, further:

      in step b), dissolving the non-water soluble organic dye with the organic solution expanded with the CF, and then, proceed with step c).

   wherein the water-soluble organic dye, the non-water soluble organic dye, the quaternary ammonium surfactant, the sterol or derivatives thereof, and the molar ratio of the quaternary ammonium surfactant molecule to sterol molecule or derivatives thereof have the same definitions as above.

12. The method according to claim 11, wherein the organic solvent is selected from a monohydric alcohol a polyhydric alcohol, a ketone, ethylenediamine, acetonitrile, ethyl acetate and mixtures thereof; and the CF is selected from selected $CO_2$, ethane, propane, a hydrochlorofluorocarbon, and a hydrofluorocarbon.

13. Use of a stable fluorescent nanovesicle according to any one of claims 1 to 10, as a fluorescent probe in an aqueous medium.

14. Use according to claim 13 for bioimaging and/or biodetection in an *in vitro* or *in vivo* method.

15. A stable colloidal dispersion comprising a plurality of stable fluorescent nanovesicles according to any one of claims 1 to 10.

## FIG 1

| -- | DLS | | NTA | |
|---|---|---|---|---|
| D, nm | 68,31 | +/- 31,55 | 93,6 | +/-29,7 |

## FIG 2

## FIG 3

# FIG 4

# FIG 5

# FIG 6

## FIG 7

## FIG 8

# FIG 9

# FIG 10

## FIG 11

# FIG 12

# FIG 13

## FIG 14

# FIG 15

Legend (top graph):
- Cascade, $\lambda_{exc}=540$
- QS-DiI $\lambda_{exc}=550$
- QS-DiD $\lambda_{exc}=600$
- QS-DiR $\lambda_{exc}=730$

Axes: Normalized intensity vs Wavelength, nm

Legend (bottom graph):
- FRET-nanovesicles, $\lambda_{exc}=490$ nm
- DiI-nanovesicles, $\lambda_{exc}=490$ nm
- DiD-nanovesicles, $\lambda_{exc}=600$ nm

Axes: Normalized intensity vs Wavelength, nm

FIG 16

Europäisches
Patentamt

European
Patent Office

Office européen
des brevets

# EUROPEAN SEARCH REPORT

Application Number

EP 16 38 2392

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/164072 A1 (LINDER CHARLES [IL] ET AL) 28 June 2012 (2012-06-28) * the whole document * * In particular: Examples 1-29; Claims 1-30. * | 1-15 | INV. A61K49/22 |
| X | FENART L ET AL: "EVALUATION OF EFFECT OF CHARGE AND LIPID COATING ON ABILITY OF 60-NM NANOPARTICLES TO CROSS AN IN VITRO MODEL OF THE BLOOD-BRAIN BARRIER", JOURNAL OF PHARMACOLOGY AND EXPERIMENTAL THERAPEUTICS, AMERICAN SOCIETY FOR PHARMACOLOGY AND EXPERIMENTAL THERAPEUTICS, US, vol. 291, no. 3, 1 January 1999 (1999-01-01), pages 1017-1022, XP000938176, ISSN: 0022-3565 * the whole document * * In particular: Title; Abstract; Materials and methods section. * | 1-10, 13-15 | |
| A | US 2012/253191 A1 (ZHENG GANG [CA] ET AL) 4 October 2012 (2012-10-04) * the whole document * | 1-15 | TECHNICAL FIELDS SEARCHED (IPC) A61K |
| A | S. GRINBERG ET AL: "Novel Cationic Amphiphilic Derivatives from Vernonia Oil: Synthesis and Self-Aggregation into Bilayer Vesicles, Nanoparticles, and DNA Complexants", LANGMUIR, vol. 21, no. 17, 1 August 2005 (2005-08-01), pages 7638-7645, XP55335415, US ISSN: 0743-7463, DOI: 10.1021/la050091j * the whole document * | 1-15 | |

The present search report has been drawn up for all claims

| Place of search | Date of completion of the search | Examiner |
|---|---|---|
| The Hague | 16 January 2017 | C.F. Angioni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 38 2392

This annex lists the patent family members relating to the patent documents cited in  the above-mentioned European search report.
The members are as contained in the European Patent Office EDP file on
The European Patent Office is in no way liable for these particulars which are merely given for the purpose of information.

16-01-2017

| Patent document cited in search report | | | Publication date | Patent family member(s) | | | Publication date |
|---|---|---|---|---|---|---|---|
| US 2012164072 | A1 | | 28-06-2012 | AU | 2010245629 | A1 | 12-01-2012 |
| | | | | AU | 2016231567 | A1 | 20-10-2016 |
| | | | | CA | 2761042 | A1 | 11-11-2010 |
| | | | | EP | 2427175 | A2 | 14-03-2012 |
| | | | | US | 2012164072 | A1 | 28-06-2012 |
| | | | | WO | 2010128504 | A2 | 11-11-2010 |
| US 2012253191 | A1 | | 04-10-2012 | CA | 2776796 | A1 | 21-04-2011 |
| | | | | CN | 102573914 | A | 11-07-2012 |
| | | | | EP | 2488206 | A1 | 22-08-2012 |
| | | | | JP | 5781518 | B2 | 24-09-2015 |
| | | | | JP | 2013507399 | A | 04-03-2013 |
| | | | | JP | 2015131841 | A | 23-07-2015 |
| | | | | US | 2012253191 | A1 | 04-10-2012 |
| | | | | WO | 2011044671 | A1 | 21-04-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0185680 A

**Non-patent literature cited in the description**

- Multifunctional Nanovesicle-bioactive conjugates prepared by a one-step scalable method using CO2-expanded solvents. Nano letters. ACS Publications, 05 July 2013
- **LIU, P. ; LIU, P. ; ZHAO, K. ; LI, L.** Photostability enhancement of azoic dyes adsorbed and intercalated into Mg-Al-layered double hydroxide. *Opt. Laser Technol.,* 2015, vol. 74, 23-28
- **GHOSH et al.** How Does the Surface Charge of Ionic Surfactant and Cholesterol Forming Vesicles Control Rotational and Translational Motion of Rhodamine 6G Perchlorate (R6G ClO4). *Langmuir,* 2015, vol. 31 (8), 2310-2320

- **TERENZIANI et al.** Dipolar versus Octupolar Triphenylamine-Based Fluorescent Organic Nanoparticles as Brilliant One- and Two-Photon Emitters for (Bio)imaging. *Small,* 2011, vol. 7 (22), 3219-3229
- **CANO-SARABIA M et al.** *Langmuir,* 2008, vol. 24, 2433-2437
- **CABRERA I et al.** *Nano Lett.,* 2013, vol. 13, 3766-3774
- **BELFIELD et al.** Photophysical and photochemical properties of 5,7-di-methoxycoumarin under one- and two-photon excitation. *J. Phys. Org. Chem.,* 2003, vol. 16, 69-78